# EUROPEAN PATENT SPECIFICATION

(11) **EP 4 614 091 B1**
(45) Date of publication and mention of the grant of the patent: **08.07.2026**
(21) Application number: 24207359.1
(22) Date of filing: 18.10.2024
(51) Int. Cl.: F25D 3/08, A61F 7/10

(54) **SELF-ABSORBENT ICE PACK**
SELBSTABSORBIERENDE EISPACKUNG
EMBALLAGE RÉFRIGÉRANT AUTO-ABSORBANT

(30) Priority: 08.10.2024 CN 202411395658
(43) Date of publication of application: 10.09.2025
(73) Proprietor: Shanghai Huizhou Industrial Co., Ltd., Shanghai (CN)
(72) Inventor: Zhang, Jun, Shanghai (CN); Ding, Yirui, Shanghai (CN); Zhang, Qiangwen, Shanghai (CN); Xu, Chengqiang, Shanghai (CN); Zhou, Musen, Shanghai (CN)
(74) Representative: Metida

(56) References cited:
- EP-B1- 0 350 377
- CN-U- 209 279 465
- CN-U- 220 077 183
- US-A1- 2014 371 828

## Description

### Technical Field

The invention relates to the technical field of ice packs, and in particular to a self-absorbent ice pack.

### Background

An ice pack is a freezing medium, which has no water pollution when thawed and melted, and can be used repeatedly under both cold and hot conditions. Its effective cold capacity is 6 times that of the same volume of ice. It can be used instead of dry ice, ice cubes, etc. The ice pack is widely used. For example, the ice pack can be used in medical treatment, such as bringing down a high fever, diminishing inflammation and relieving pain, and adjuvant physiotherapy, such as cold compress for beauty treatment, sprain relief, hemostasis, purulency treatment and skin care. The ice pack can be used for refrigerated transportation of various cryoprotectant agents, and long-distance refrigerated transportation of solder paste, poultry drugs, medicines, plasma, vaccines, aquatic products, poultry, ornamental fish and fresh food in foreign trade. The ice pack can also be used for injuries, sprains and falls of sports athletes during training and competitions. Moreover, the ice pack can be used for cold storage for power saving in daily life, and for maintaining low temperature, keeping food fresh and cooling drinks when the refrigerator is out of power or during travel.

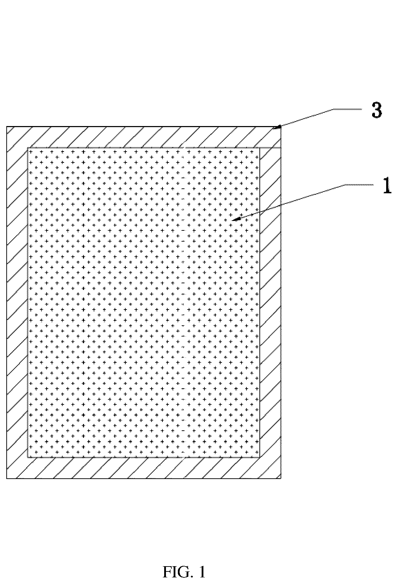

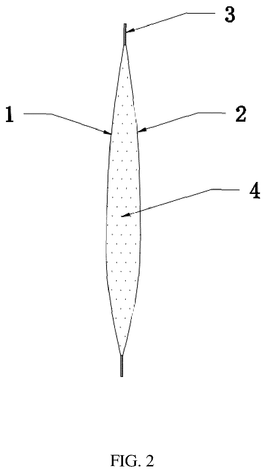

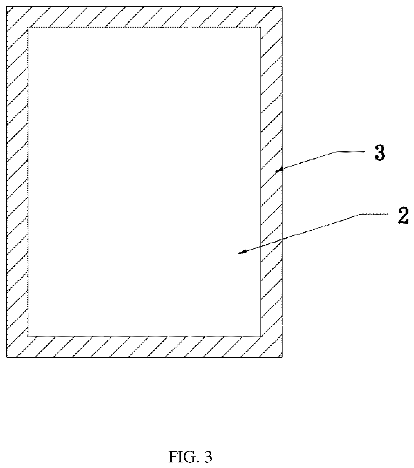

At present, the commonly used ice packs are as follows: FIG. 1 to FIG. 3 show an existing ice pack I. The ice pack I has a non-woven fabric surface 1 and a composite plastic layer 2. Edges of the non-woven fabric surface 1 and the composite plastic layer 2 are hot-pressed to form a heat-sealed edge I 3, which is filled with absorbent resin granules 4 inside. When in use, the ice pack I is put into water, and water permeates to the inside through the non-woven fabric surface 1. The water comes into contact with the absorbent resin granules 4, and the absorbent resin granules 4 absorb the water and swell.

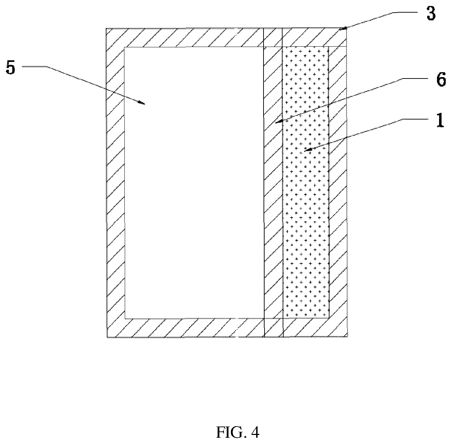

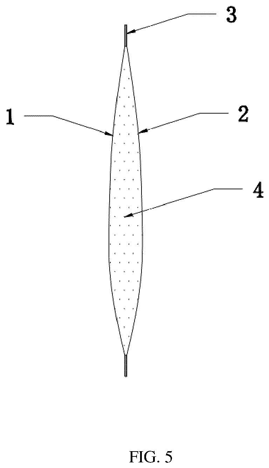

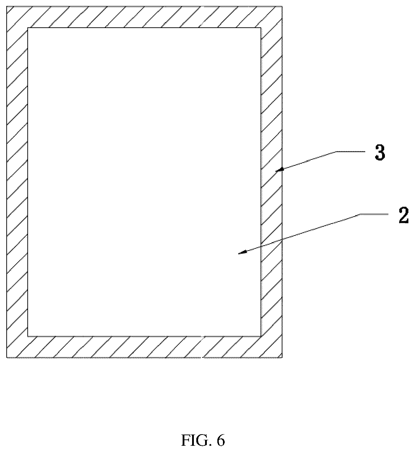

FIG. 4 to FIG. 6 show an existing ice pack II. One side edge of a non-woven fabric surface 1 is connected to a semi-composite plastic strip 5 by hot pressing. In this way, when the ice pack II is immersed in water, the water enters the inside through the non-woven fabric surface 1. The semi-composite plastic strip 5 takes up most of the area. When the ice pack II swells, the semi-composite plastic strip 5 protrudes. When adjacent ice packs II are in contact, the semi-composite plastic strip 5 is less likely to retain water due to its smooth surface, and therefore, it is less susceptibility to icing and sticking.

There is also an ice pack III (not shown) formed by hot-pressing two composite plastic layers 2, and micropores are made in surfaces of the composite plastic layers 2 by laser drilling.

The above ice packs have the following problems: The non-woven fabric surface 1 of the ice pack I has a porous surface. When the ice pack I is squeezed by water, a middle part of the non-woven fabric surface 1 protrudes outwards. Since the non-woven fabric surface 1 is water-permeable, there is water residing on the surface of the non-woven fabric surface 1. In this case, when the ice packs I are frozen, the non-woven fabric surfaces 1 of the ice pack I are prone to freeze and adhere to each other. If the ice packs I are forced apart, the non-woven fabric surfaces 1 are prone to tear.

When the ice pack II is immersed in water, since the non-woven fabric surface 1 is located on one side of the semi-composite plastic strip 5, both water inflowing and air evacuation are realized through the non-woven fabric surface 1, causing low water inflowing efficiency.

The ice pack III has fewer micropores than the pores in the non-woven fabric surface 1, which leads to slow water inflowing. If a pore size of the micropores is increased, the absorbent resin granules 4 will easily ooze out.

EP 0350377B1 disclose a cooling pack according to the preamble of claim 1, including at least one coolant carrier for keeping coolant held thereon and a container adapted to enclose the coolant carrier. The coolant carrier consists of liquid absorbent substrate and liquid absorbent polymer particles. The container has at least a pair of flat layers opposite each other which are adapted to introduce liquid into the container. The cooling pack can provide beneficial features such as avoid exudation of liquid absorbent polymer particles, to introduce liquid into the container in a short period of time, and to obtain non-sticking of the container refrigerated condition.

CN 220077183U discloses a quick water absorption ice bag, which includes a first waterproof layer, a bag bottom and water absorption particles, the first waterproof layer is fixedly connected with the four edges of the bag bottom, a containing cavity is formed between the first waterproof layer and the bag bottom, the water absorption particles are placed in the containing cavity, and the quick water absorption ice bag further includes a covering layer.

CN 209279465U discloses a self-water-absorption type ice bag. The ice bag includes non-woven fabric that includes a square non-woven fabric layer, a square first plastic film layer and a water-absorbing coolant.

### Summary

The present invention is defined in the independent claims. An objective of the invention is to provide a self-absorbent ice pack to solve the problems in the Background.

In order to achieve the above objective, the invention provides the following technical solution: A self-absorbent ice pack includes:
an outer bag body and absorbent resin granules filling an inside of the outer bag body,
where the outer bag body includes a composite plastic layer and a water-permeable layer, an edge of the water-permeable layer and an edge of the composite plastic layer being hot-pressed to form a heat-sealed edge I; and
the water-permeable layer includes a non-woven fabric surface and a semi-composite plastic strip arranged in a middle part of the non-woven fabric surface, and a joint between the semi-composite plastic strip and the non-woven fabric surface is hot-pressed to form a heat-sealed edge II.

The composite plastic layer is made of a PE material.

Preferably, a number of the non-woven fabric surfaces is set to one, and the semi-composite plastic strip is hot-pressed to the middle part of the non-woven fabric surface.

Preferably, a number of the non-woven fabric surfaces is set to two, and a hot pressing space is arranged between the two non-woven fabric surfaces, the semi-composite plastic strip being located in the hot pressing space, and edges on two sides of the semi-composite plastic strip being connected to corresponding edges of the non-woven fabric surfaces on two sides by hot pressing.

A width of the non-woven fabric surface between the heat-sealed edge I and the semi-composite plastic strip is 2.8 cm-3.2 cm.

The composite plastic layer and the semi-composite plastic strip are made of a same material.

Preferably, a one-cell water storage region or a multi-cell water storage region is formed between the composite plastic layer and the water-permeable layer; and when the multi-cell water storage region is formed between the composite plastic layer and the water-permeable layer, a plurality of heat-sealed edges III intersecting transversely and longitudinally are formed between the composite plastic layer and the water-permeable layer by hot pressing, and a region formed between the heat-sealed edges III is the multi-cell water storage region.

The self-absorbent ice pack is specifically prepared by the following steps:
S1: setting sizes of the composite plastic layer and the water-permeable layer, and cutting the composite plastic layer;
S2: hot-pressing the non-woven fabric surface and two sides of the semi-composite plastic strip, then cutting the non-woven fabric surface and the semi-composite plastic strip as a whole according to a size to form the water-permeable layer, a size of the water-permeable layer being the same as that of the composite plastic layer; and
S3: attaching the composite plastic layer to the water-permeable layer, hot-pressing three edges of the composite plastic layer and the water-permeable layer to form an ice pack with an opening, filling the ice pack with the absorbent resin granules through the opening, and sealing the opening of the ice pack by hot pressing.

Compared with the prior art, the invention has the following advantages:
In this solution, the semi-composite plastic strip is arranged in the middle part of the water-permeable layer, the non-woven fabric surfaces on the two sides of the semi-composite plastic strip are respectively used for water inflowing and air evacuation, or water inflowing and air evacuation are carried out through the non-woven fabric surfaces on the two sides at the same time, which improves the water inflowing efficiency and prevents the absorbent resin granules inside from oozing out.

When water enters the inside of the ice pack and the ice pack swells, the semi-composite plastic strip protrudes. The ice packs contact mainly at the protruding semi-composite plastic strips. Thus, the surface is less likely to retain water or freeze and stick.

### Brief Description of Figures

FIG. 1 is a schematic structural view of a non-woven fabric surface of an existing ice pack I;
FIG. 2 is a sectional view of the existing ice pack I;
FIG. 3 is a schematic structural view of a composite plastic layer of the existing ice pack I;
FIG. 4 is a schematic structural view of a non-woven fabric surface and a semi-composite plastic strip of an existing ice pack II;
FIG. 5 is a sectional view of the existing ice pack II;
FIG. 6 is a schematic structural view of a composite plastic layer of the existing ice pack II;
FIG. 7 is a schematic structural front view of the invention;
FIG. 8 is a sectional view of the invention;
FIG. 9 is a back view of the invention;
FIG. 10 is a schematic structural view of a non-woven fabric surface and a semi-composite plastic strip of the invention connected by a first method; and
FIG. 11 is a schematic structural view of the non-woven fabric surface and the semi-composite plastic strip of the invention connected by a second method.

In the figures: 1. non-woven fabric surface; 2, composite plastic layer; 3, heat-sealed edge I; 4, absorbent resin granule; 5, semi-composite plastic strip; 6, heat-sealed edge II; and 7, hot pressing space.

### Detailed Description

The technical solutions in embodiments of the invention will be clearly and completely described below with reference to the accompanying drawings in the embodiments of the invention. It is apparent that the described embodiments are only a part, rather than all of the embodiments of the invention. All other embodiments obtained by those of ordinary skill in the art based on the embodiments of the invention without creative work are within the protection scope of the invention.

In the description of the invention, it should be understood that, the orientation or positional relationship indicated by the term "upper", "lower", "front", "rear", "left", "right", "top", "bottom", "inner", "outer" or the like is based on the orientation or positional relationship shown in the accompanying drawings. This is only for the convenience of describing the invention and simplifying the description, and does not indicate or imply that a device or element must have a specific orientation, or be constructed and operated in a specific orientation, and therefore cannot be understood as a limitation of the invention.

### Embodiment I:

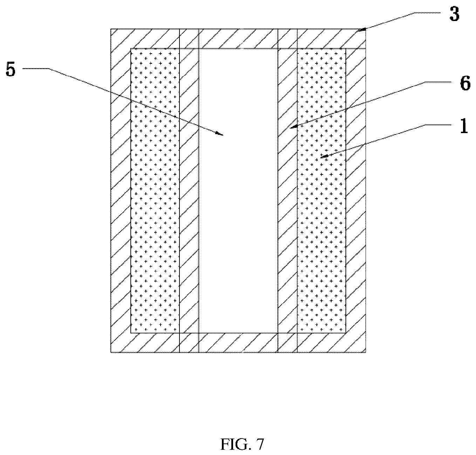

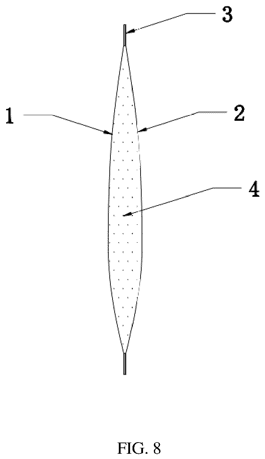

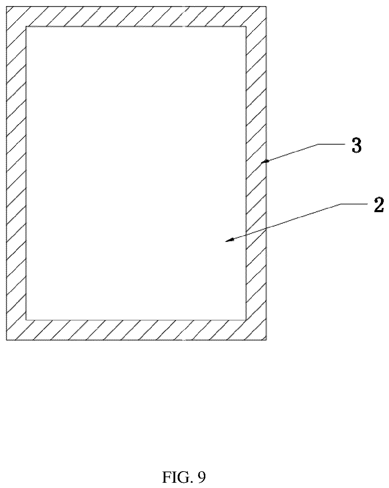

Referring to FIG. 7 to FIG. 11, the invention provides a technical solution: A self-absorbent ice pack includes:
an outer bag body and absorbent resin granules 4 filling an inside of the outer bag body.

The outer bag body includes a composite plastic layer 2 and a water-permeable layer. An edge of the water-permeable layer and an edge of the composite plastic layer 2 are hot-pressed to form a heat-sealed edge I 3. The water-permeable layer includes a non-woven fabric surface 1 and a semi-composite plastic strip 5 arranged in a middle part of the non-woven fabric surface 1. A joint between the semi-composite plastic strip 5 and the non-woven fabric surface 1 is hot-pressed to form a heat-sealed edge II 6.

Analysis for the above contents: When in actual use, the ice pack is put in water, with the non-woven fabric surface 1 on one side below the water surface and the non-woven fabric surface 1 on the other side above the water surface. In this way, the non-woven fabric surface 1 below the water surface can be used for water inflowing, and the non-woven fabric surface 1 on the other side can be used for air evacuation, so that air and water flow along a single direction without hindering each other. Thereby, the overall water inflowing efficiency is higher. When enough water enters the ice pack, the whole ice pack swells up. Since the semi-composite plastic strip 5, which is located in the middle part, protrudes the highest, the semi-composite plastic strips 5 of the adjacent ice packs are most likely to be close to each other. Due to material characteristics of the semi-composite plastic strip 5, its surface is smooth and thus it is not easy to freeze and stick between the adjacent ice packs.

### Embodiment II:

Referring to FIG. 7 to FIG. 11, the invention provides a technical solution based on Embodiment I: The composite plastic layer 2 is made of a PE material. The composite plastic layer 2 and the semi-composite plastic strip 5 are made of a same material.

Analysis for the above contents: The PE material has a smooth surface and good toughness, and is not easy to get wet.

### Embodiment III:

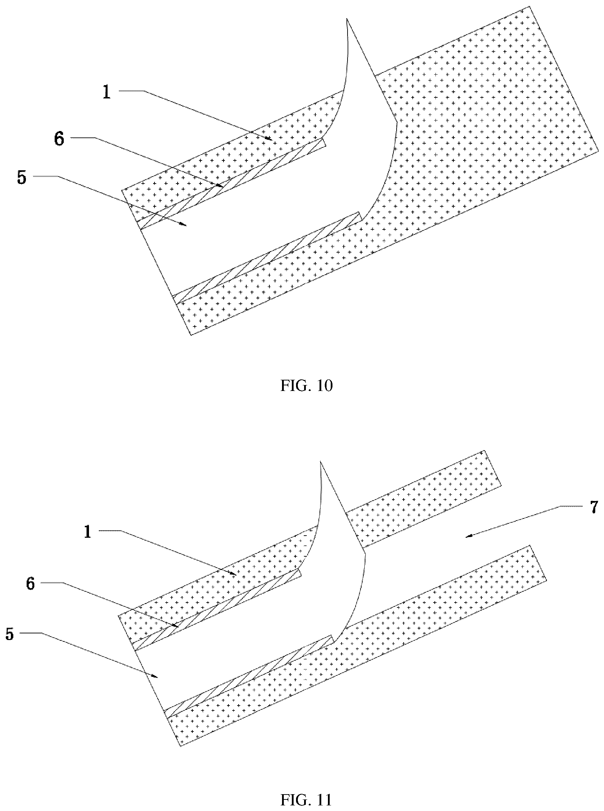

Referring to FIG. 7 to FIG. 11, the invention provides a technical solution based on Embodiment I: The non-woven fabric surface 1 and the semi-composite plastic strip 5 are connected by the following two methods:
First method: As shown in FIG. 10, a number of the non-woven fabric surfaces 1 is set to one, and the semi-composite plastic strip 5 is hot-pressed to the middle part of the non-woven fabric surface 1.
Second method: As shown in FIG. 11, a number of the non-woven fabric surfaces 1 is set to two, and a hot pressing space 7 is arranged between the two non-woven fabric surfaces 1. The semi-composite plastic strip 5 is located in the hot pressing space 7, and edges on two sides of the semi-composite plastic strip 5 are connected to corresponding edges of the non-woven fabric surfaces 1 on two sides by hot pressing.

Analysis for the above contents: Both the two methods for connecting the non-woven fabric surface 1 and the semi-composite plastic strip 5 can be achieved. The second method is relatively material-saving, and the first method is relatively simple to implement.

### Embodiment IV:

Referring to FIG. 7 to FIG. 11, the invention provides a technical solution based on Embodiment III: A width of the non-woven fabric surface 1 between the heat-sealed edge I 3 and the semi-composite plastic strip 5 is 2.8 cm-3.2 cm.

Analysis for the above contents: The width of the non-woven fabric surface 1 between the heat-sealed edge I 3 and the semi-composite plastic strip 5 is preferably 3 cm, which can ensure the water absorption efficiency and avoid adhesion.

### Embodiment V:

Referring to FIG. 7 to FIG. 11, the invention provides a technical solution based on Embodiment I: A one-cell water storage region or a multi-cell water storage region is formed between the composite plastic layer 2 and the water-permeable layer. When the multi-cell water storage region is formed between the composite plastic layer 2 and the water-permeable layer, a plurality of heat-sealed edges III intersecting transversely and longitudinally are formed between the composite plastic layer 2 and the water-permeable layer by hot pressing, and a region formed between the heat-sealed edges III is the multi-cell water storage region.

Analysis for the above contents: When the whole ice pack is made with a large size, in order to make the absorbent resin granules 4 dispersed uniformly, the inside of the ice pack is divided into a plurality of cells (water storage region), so that the absorbent resin granules 4 can be dispersed in the plurality of cells. The absorbent resin granules in the plurality of cells can absorb water separately. Moreover, the cells are isolated by the heat-sealed edges III, and thus, are less prone to affect each other.

### Embodiment VI:

Referring to FIG. 7 to FIG. 11, the invention provides a technical solution based on Embodiment I: The novel self-absorbent ice pack is specifically prepared by the following steps:
S1: setting sizes of the composite plastic layer 2 and the water-permeable layer, and cutting the composite plastic layer 2;
S2: hot-pressing the non-woven fabric surface 1 and two sides of the semi-composite plastic strip 5, then cutting the non-woven fabric surface 1 and the semi-composite plastic strip 5 as a whole according to a size to form the water-permeable layer, a size of the water-permeable layer being the same as that of the composite plastic layer 2; and
S3: attaching the composite plastic layer 2 to the water-permeable layer, hot-pressing three edges of the composite plastic layer 2 and the water-permeable layer to form an ice pack with an opening, filling the ice pack with the absorbent resin granules 4 through the opening, and sealing the opening of the ice pack by hot pressing.

## Claims

1. A self-absorbent ice pack, comprising:
an outer bag body and absorbent resin granules (4) filling an inside of the outer bag body,
wherein the outer bag body comprises a composite plastic layer (2) and a water-permeable layer, an edge of the water-permeable layer and an edge of the composite plastic layer (2) being hot-pressed to form a heat-sealed edge I (3); and **characterized in that**
the water-permeable layer comprises a non-woven fabric surface (1) and a semi-composite plastic strip (5) arranged in a middle part of the non-woven fabric surface (1), and a joint between the semi-composite plastic strip (5) and the non-woven fabric surface (1) is hot-pressed to form a heat-sealed edge II (6);
wherein a width of the non-woven fabric surface (1) between the heat-sealed edge I (3) and the semi-composite plastic strip (5) is 2.8 cm-3.2 cm;
wherein the composite plastic layer (2) and the semi-composite plastic strip (5) are made of PE material.

2. The self-absorbent ice pack according to claim 1, **characterized in that** a number of the non-woven fabric surfaces (1) is set to one, and the semi-composite plastic strip (5) is hot-pressed to the middle part of the non-woven fabric surface (1).

3. The self-absorbent ice pack according to claim 1, **characterized in that** a number of the non-woven fabric surfaces (1) is set to two, and a hot pressing space (7) is arranged between the two non-woven fabric surfaces (1), the semi-composite plastic strip (5) being located in the hot pressing space (7), and edges on two sides of the semi-composite plastic strip (5) being connected to corresponding edges of the non-woven fabric surfaces (1) on two sides by hot pressing.

4. The self-absorbent ice pack according to claim 1, **characterized in that** a one-cell water storage region or a multi-cell water storage region is formed between the composite plastic layer (2) and the water-permeable layer; and when the multi-cell water storage region is formed between the composite plastic layer (2) and the water-permeable layer, a plurality of heat-sealed edges III intersecting transversely and longitudinally are formed between the composite plastic layer (2) and the water-permeable layer by hot pressing, and a region formed between the heat-sealed edges III is the multi-cell water storage region.

5. A method for producing the self-absorbent ice pack according to claim 1, **characterized in that** the self-absorbent ice pack is specifically prepared by the following steps:
S1: setting sizes of the composite plastic layer (2) and the water-permeable layer, and cutting the composite plastic layer (2);
S2: hot-pressing the non-woven fabric surface (1) and two sides of the semi-composite plastic strip (5), then cutting the non-woven fabric surface (1) and the semi-composite plastic strip (5) as a whole according to a size to form the water-permeable layer, a size of the water-permeable layer being the same as that of the composite plastic layer (2); wherein the width of the non-woven fabric surface (1) between the heat-sealed edge I (3) and the semi-composite plastic strip (5) is 2.8 cm to 3.2 cm, and wherein the composite plastic layer (2) and the semi-composite plastic strip (5) are made of PE material; and
S3: attaching the composite plastic layer (2) to the water-permeable layer, hot-pressing three edges of the composite plastic layer (2) and the water-permeable layer to form an ice pack with an opening, filling the ice pack with the absorbent resin granules (4) through the opening, and sealing the opening of the ice pack by hot pressing.

## Patentansprüche

1. Eine selbstabsorbierende Eispackung, umfassend:
einen äußeren Beutelkörper und absorbierende Harzkörnchen (4), die ein Inneres des äußeren Beutelkörpers füllen,
wobei der äußere Beutelkörper eine Verbundkunststoffschicht (2) und eine wasserdurchlässige Schicht umfasst, wobei ein Rand der wasserdurchlässigen Schicht und ein Rand der Verbundkunststoffschicht (2) heißgepresst sind, um einen heißgesiegelten Rand I (3) zu bilden; und
**dadurch gekennzeichnet, dass**
die wasserdurchlässige Schicht eine Vliesstoffoberfläche (1) und einen Halbverbund-Kunststoffstreifen (5) umfasst, der in einem mittleren Teil der Vliesstoffoberfläche (1) angeordnet ist, und eine Verbindung zwischen dem Halbverbund-Kunststoffstreifen (5) und der Vliesstoffoberfläche (1) heißgepresst ist, um einen heißgesiegelten Rand II (6) zu bilden;
wobei eine Breite der Vliesstoffoberfläche (1) zwischen dem heißgesiegelten Rand I (3) und dem Halbverbund-Kunststoffstreifen (5) 2.8 cm bis 3.2 cm beträgt;
wobei die Verbundkunststoffschicht (2) und der Halbverbund-Kunststoffstreifen (5) aus PE-Material hergestellt sind.

2. Die selbstabsorbierende Eispackung nach Anspruch 1, **dadurch gekennzeichnet, dass** eine Anzahl der Vliesstoffoberflächen (1) auf eins festgelegt ist und der Halbverbund-Kunststoffstreifen (5) auf den mittleren Teil der Vliesstoffoberfläche (1) heißgepresst ist.

3. Die selbstabsorbierende Eispackung nach Anspruch 1, **dadurch gekennzeichnet, dass** eine Anzahl der Vliesstoffoberflächen (1) auf zwei festgelegt ist und ein Heißpressraum (7) zwischen den beiden Vliesstoffoberflächen (1) angeordnet ist, wobei sich der Halbverbund-Kunststoffstreifen (5) in dem Heißpressraum (7) befindet und Ränder auf zwei Seiten des Halbverbund-Kunststoffstreifens (5) durch Heißpressen mit entsprechenden Rändern der Vliesstoffoberflächen (1) auf zwei Seiten verbunden sind.

4. Die selbstabsorbierende Eispackung nach Anspruch 1, **dadurch gekennzeichnet, dass** ein einzelliger Wasserspeicherbereich oder ein mehrzelliger Wasserspeicherbereich zwischen der Verbundkunststoffschicht (2) und der wasserdurchlässigen Schicht gebildet ist; und wenn der mehrzellige Wasserspeicherbereich zwischen der Verbundkunststoffschicht (2) und der wasserdurchlässigen Schicht gebildet ist, eine Vielzahl von transversal und longitudinal kreuzenden heißgesiegelten Rändern III zwischen der Verbundkunststoffschicht (2) und der wasserdurchlässigen Schicht durch Heißpressen gebildet ist, und ein zwischen den heißgesiegelten Rändern III gebildeter Bereich der mehrzellige Wasserspeicherbereich ist.

5. Ein Verfahren zur Herstellung der selbstabsorbierenden Eispackung nach Anspruch 1, **dadurch gekennzeichnet, dass** die selbstabsorbierende Eispackung spezifisch durch die folgenden Schritte hergestellt wird:
S1: Einstellen von Größen der Verbundkunststoffschicht (2) und der wasserdurchlässigen Schicht und Zuschneiden der Verbundkunststoffschicht (2);
S2: Heißpressen der Vliesstoffoberfläche (1) und von zwei Seiten des Halbverbund-Kunststoffstreifens (5), dann Zuschneiden der Vliesstoffoberfläche (1) und des Halbverbund-Kunststoffstreifens (5) als Ganzes gemäß einer Größe, um die wasserdurchlässige Schicht zu bilden, wobei eine Größe der wasserdurchlässigen Schicht dieselbe wie die der Verbundkunststoffschicht (2) ist; wobei die Breite der Vliesstoffoberfläche (1) zwischen dem heißgesiegelten Rand I (3) und dem Halbverbund-Kunststoffstreifen (5) 2.8 cm bis 3.2 cm beträgt und wobei die Verbundkunststoffschicht (2) und der Halbverbund-Kunststoffstreifen (5) aus PE-Material hergestellt sind; und
S3: Anbringen der Verbundkunststoffschicht (2) an der wasserdurchlässigen Schicht, Heißpressen von drei Rändern der Verbundkunststoffschicht (2) und der wasserdurchlässigen Schicht, um eine Eispackung mit einer Öffnung zu bilden, Füllen der Eispackung mit den absorbierenden Harzkörnchen (4) durch die Öffnung und Versiegeln der Öffnung der Eispackung durch Heißpressen.

## Revendications

1. Un emballage réfrigérant auto-absorbant, comprenant:
un corps de sac extérieur et des granulés de résine absorbante (4) remplissant un intérieur du corps de sac extérieur,
dans lequel le corps de sac extérieur comprend une couche de plastique composite (2) et une couche perméable à l'eau, un bord de la couche perméable à l'eau et un bord de la couche de plastique composite (2) étant pressés à chaud pour former un bord thermoscellé I (3) ; et
**caractérisé en ce que**
la couche perméable à l'eau comprend une surface en tissu non tissé (1) et une bande en plastique semi-composite (5) agencée dans une partie centrale de la surface en tissu non tissé (1), et une jonction entre la bande en plastique semi-composite (5) et la surface en tissu non tissé (1) est pressée à chaud pour former un bord thermoscellé II (6) ;
dans lequel une largeur de la surface en tissu non tissé (1) entre le bord thermoscellé I (3) et la bande en plastique semi-composite (5) est de 2.8 cm à 3.2 cm ;
dans lequel la couche de plastique composite (2) et la bande en plastique semi-composite (5) sont constituées de matériau PE.

2. L'emballage réfrigérant auto-absorbant selon la revendication 1, **caractérisé en ce qu'**un nombre des surfaces en tissu non tissé (1) est fixé à un, et la bande en plastique semi-composite (5) est pressée à chaud sur la partie centrale de la surface en tissu non tissé (1).

3. L'emballage réfrigérant auto-absorbant selon la revendication 1, **caractérisé en ce qu'**un nombre des surfaces en tissu non tissé (1) est fixé à deux, et un espace de pressage à chaud (7) est agencé entre les deux surfaces en tissu non tissé (1), la bande en plastique semi-composite (5) étant située dans l'espace de pressage à chaud (7), et des bords sur deux côtés de la bande en plastique semi-composite (5) étant reliés à des bords correspondants des surfaces en tissu non tissé (1) sur deux côtés par pressage à chaud.

4. L'emballage réfrigérant auto-absorbant selon la revendication 1, **caractérisé en ce qu'**une région de stockage d'eau à une cellule ou une région de stockage d'eau à cellules multiples est formée entre la couche de plastique composite (2) et la couche perméable à l'eau ; et lorsque la région de stockage d'eau à cellules multiples est formée entre la couche de plastique composite (2) et la couche perméable à l'eau, une pluralité de bords thermoscellés III s'entrecroisant transversalement et longitudinalement sont formés entre la couche de plastique composite (2) et la couche perméable à l'eau par pressage à chaud, et une région formée entre les bords thermoscellés III est la région de stockage d'eau à cellules multiples.

5. Un procédé de fabrication de l'emballage réfrigérant auto-absorbant selon la revendication 1, **caractérisé en ce que** l'emballage réfrigérant auto-absorbant est spécifiquement préparé par les étapes suivantes:
S1: le réglage des tailles de la couche de plastique composite (2) et de la couche perméable à l'eau, et la découpe de la couche de plastique composite (2) ;
S2: le pressage à chaud de la surface en tissu non tissé (1) et de deux côtés de la bande en plastique semi-composite (5), puis la découpe de la surface en tissu non tissé (1) et de la bande en plastique semi-composite (5) dans leur ensemble selon une taille pour former la couche perméable à l'eau, une taille de la couche perméable à l'eau étant la même que celle de la couche de plastique composite (2) ; dans lequel la largeur de la surface en tissu non tissé (1) entre le bord thermoscellé I (3) et la bande en plastique semi-composite (5) est de 2.8 cm à 3.2 cm, et dans lequel la couche de plastique composite (2) et la bande en plastique semi-composite (5) sont constituées de matériau PE ; et
S3: la fixation de la couche de plastique composite (2) à la couche perméable à l'eau, le pressage à chaud de trois bords de la couche de plastique composite (2) et de la couche perméable à l'eau pour former un emballage réfrigérant avec une ouverture, le remplissage de l'emballage réfrigérant avec les granulés de résine absorbante (4) à travers l'ouverture, et le scellement de l'ouverture de l'emballage réfrigérant par pressage à chaud.
